# EUROPEAN PATENT SPECIFICATION

(11) **EP 1 435 886 B1**
(45) Date of publication and mention of the grant of the patent: **18.02.2009**
(21) Application number: 02757965.5
(22) Date of filing: 23.08.2002
(51) Int. Cl.: A61F 9/007

(54) **SURGICAL VACUUM SEALING DEVICE**
CHIRURGISCHE VAKUUMABDICHTVORRICHTUNG
DISPOSITIF CHIRURGICAL DE FERMETURE ETANCHE SOUS VIDE

(30) Priority: 11.09.2001 AU PR770901; 31.01.2002 AU PS033602; 22.04.2002 AU PS190102
(43) Date of publication of application: 14.07.2004
(73) Proprietor: Neilson, Geoffrey James, Epping, NSW 2121 (AU); Maloof, Anthony, Kingsford, New South Wales 2032 (AU)
(72) Inventor: Neilson, Geoffrey James, Epping, NSW 2121 (AU); Maloof, Anthony, Kingsford, New South Wales 2032 (AU)
(74) Representative: Ablett, Graham Keith
(86) International application number: PCT/AU2002/001147
(87) International publication number: WO 2003/022191

(56) References cited:
- WO-A-00/74574
- WO-A-02/43632
- WO-A1-00/74574
- WO-A1-02/43632
- US-A- 4 796 623
- US-A- 5 727 569
- US-A- 5 727 569
- US-A- 5 772 675
- US-A- 6 042 539
- DATABASE WPI Week 198419, Derwent Publications Ltd., London, GB; Class P34, AN 1984-119809, XP002992599 & SU 1 034 746 A (AS UKR PHYSICS INST) 15 August 1983

## Description

The invention relates to vacuum sealing device and more particularly to a vacuum sealing device enabling the delivery of solutions to body surfaces or cavities.

It is sometimes desirable, during the course of surgery, to isolate one part of the body from the rest. For example, it may be desirable to administer a chemically active or toxic substance to one part of the body without that substance contacting other parts of the body. In other instances, it may be advantageous to prevent secretions, infectious materials or contaminants from spreading outside of the locality of the area which is being operated on. The present invention addresses these issues and illustrates the benefits of isolating a surgical site by referring to the specific example of cataract surgery.

During cataract surgery, the human lens is removed from within the lens capsule and replaced by an artificial lens. This is performed by tearing a small hole in the interior capsule (a capsulorhexis) and then destroying and removing the human lens by phacoemulsification. However, lens cortex and epithelial cells remain following the lens removal. Irrigation/aspiration is routinely used to remove the visible cortex remnants. It is unreasonable to expect all lens epithelial cells (LECs) which are bound to both the anterior and posterior capsule to be removed by this method.

LECs which remain within the capsule have been shown to mutate and grow over the posterior surface of the implanted intra-ocular lens (IOL) thus causing posterior capsule opacification (PCO). This complication of cataract surgery has historically occurred at a rate as high as 30%, however recent IOL designs have reduced this to around 2-5% at 2 years. It remains unclear what the longer term rates of PCO with these IOLs will be.

The current treatment for PCO is a posterior capsulotomy using a Yag laser. Although the complication associated with this procedure is small, the cost is significant and there is a risk of retinal detachment.

Current methods for reducing the rate of PCO include IOL design. It has been shown that a lens with sharp edges causes a barrier to LEC growth. However, lens capsule fibrosis occurs and these IOLs have been shown to cause vision problems particularly at night due to reflections from these edges.

It has been proposed that cytotoxic chemicals can be used to destroy these epithelial cells, however, there is a risk that these chemicals damage other intraocular structures.

Research is currently underway into using accommodating IOLs, and clear lens extraction for the correction of refractive errors. However, for these technologies to be successful chronically, the lens capsule must remain flexible and free of fibrosis.

Therefore, there is still a need for a device which overcomes the current problems associated with adequate capsule cleaning and LEC removal.

US-A-5 772 675 discloses a device comprising an annular ring shaped channel having an aperture therethrough. The channel is open at a lower edge with a port opens into it. The device is located over an eye so that the open lower edge is located over the cornea and then a vacuum is applied to the port so that a suction force is applied between the channel and the eye. In this way, the eye is held in a constant and fixed position with the cornea protruding through the aperture defined by the channel so that surgery can then be performed on the cornea.

WO 00/74574 discloses a stabilising device comprising a rod like instrument terminating in an elastomeric suction channel. Once the suction channel is inserted into an interior body cavity, it is transformed into a colied form for attachment to the surface of the internal treament site by introduction of a partial vacuum into the suction channel while the suction channel is held against the surface of the site. Thus, the device can temporarily and remotely hold a local area of tissue in an immobile manner.

According to the present invention there is provided a surgical vacuum sealing device comprising one or more suction rings; characterised in that the device further comprises a flexible sleeve extending to a distal end to form a flexible platter with the said one or more suction rings in an underside thereof for sealing to a body to define an enclosed surgical area, the sleeve having a suction tube for communicating a suction or pressure to the one or more suction rings, and having a tube which opens into the underside of the platter and includes an access opening for entry of surgical instruments.

### BRIEF DESCRIPTION OF DRAWINGS

Fig. 1 is a schematic cross section of the present invention as applied to cataract surgery,
Fig. 2 is a schematic top plan view of the device depicted in Fig. 1.
Figs. 3a - 3d are schematic cross sections of a sleeve of the type depicted in Figs. 1 and 2,
Fig. 4 is a schematic top plan view of an alternative sleeve throat,
Fig. 5 is a schematic cross section showing an insertion rod,
Fig. 6 is a perspective view of a capsule sealing device according to the teachings of the present invention,
Fig. 7 is a top plan view of the device depicted in Fig. 6,
Fig. 8 is a cross-section through line A-A of Fig. 7,
Fig 9 is a cross-section through line B-B of Fig. 7,
Fig. 10 is a detail of the seal depicted in Fig. 3,
Fig. 11 is a view similar to Fig. 10 of a modified form of seal,
Fig. 12 is a schematic cross sectional view of a first embodiment of the present invention,
Fig. 13 is a schematic cross sectional diagram illustrating a second embodiment of the present invention,
Fig. 14 is a schematic cross sectional diagram illustrating a third embodiment of the present invention,
Fig. 15 is a schematic cross sectional diagram illustrating a fourth embodiment of the present invention,
Fig. 16 is a schematic cross sectional diagram illustrating a flange whose upper surface is blended into a manifold,
Fig. 17 is a cross section through lines 6-6 of Fig. 14,
Fig. 18 is a schematic cross sectional view illustrating a complex curvature and an outer peripheral vacuum channel together with a pair of secondary seals which occupy a space within the outer peripheral seal, and
Fig. 19 is an elevation of a vacuum sealing device which is formed such that the peripheral vacuum seal departs from a two dimensional plane.

### MODES FOR CARRYING OUT THE INVENTION

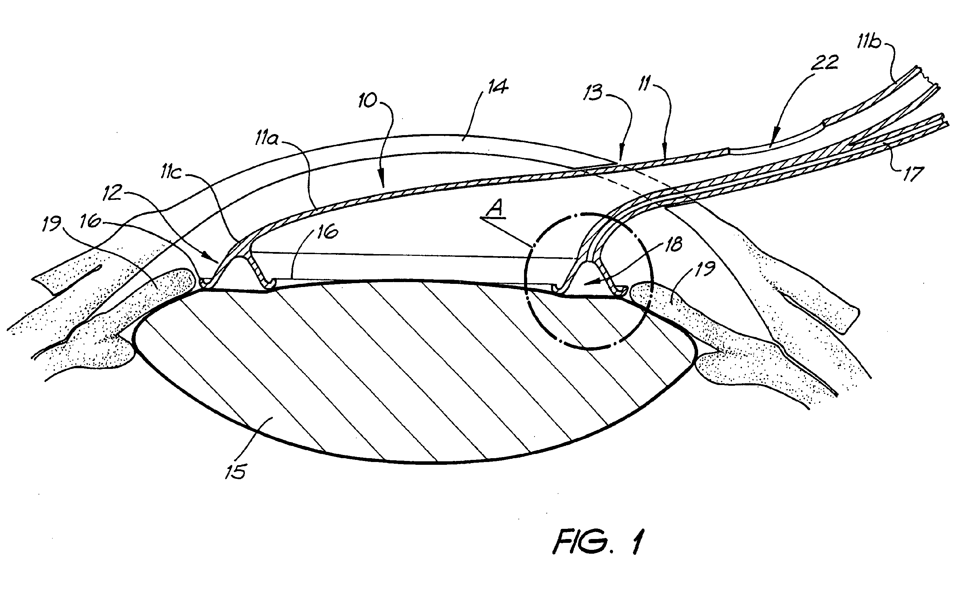

As shown in Fig. 1, a device according to one embodiment of the present invention comprises a flexible sleeve 11 having a distal extremity 11 c which terminates in a vacuum platter having a suction ring 12 around its periphery on its underside which operates as a seal. In those embodiments intended for eye surgery where the device is sealed onto a lens capsule, the device is preferably foldable and flexible enough to be folded or rolled into a conformation for insertion through a corneal incision and the vacuum platter is sized to surround the capsulorhexis so as to provide a seal. The suction ring 12 has an inverted "U" shaped cross section. The sleeve 11 and suction ring 12 are preferably made from silicone or polyurethane or other soft and flexible materials. The internal surface of the sleeve may be coated with a slippery substance such as a hydrogel. The distal portion 11 a of the sleeve 11 and suction ring 12 must be small enough and flexible enough to pass through the incision 13 in the cornea 14.

The suction ring 12 couples the sleeve 11 to the lens capsule 15. Accordingly, the distal edges 16 of the suction ring 12 are preferably blunted, radiused or otherwise enlarged to increase the contact area with the lens capsule and accordingly reduced the contact stress. The sleeve 11 incorporates or has attached to it a flexible suction tube 17. The flexible suction tube 17 communicates a suction or pressure from outside the eye to the interior 18 of the suction ring 12. The tube 17 is joined to the sleeve 11 or is formed integrally with it. It may be interior or exterior to the sleeve wall. The entire suction ring fits within the opening of the iris 19.

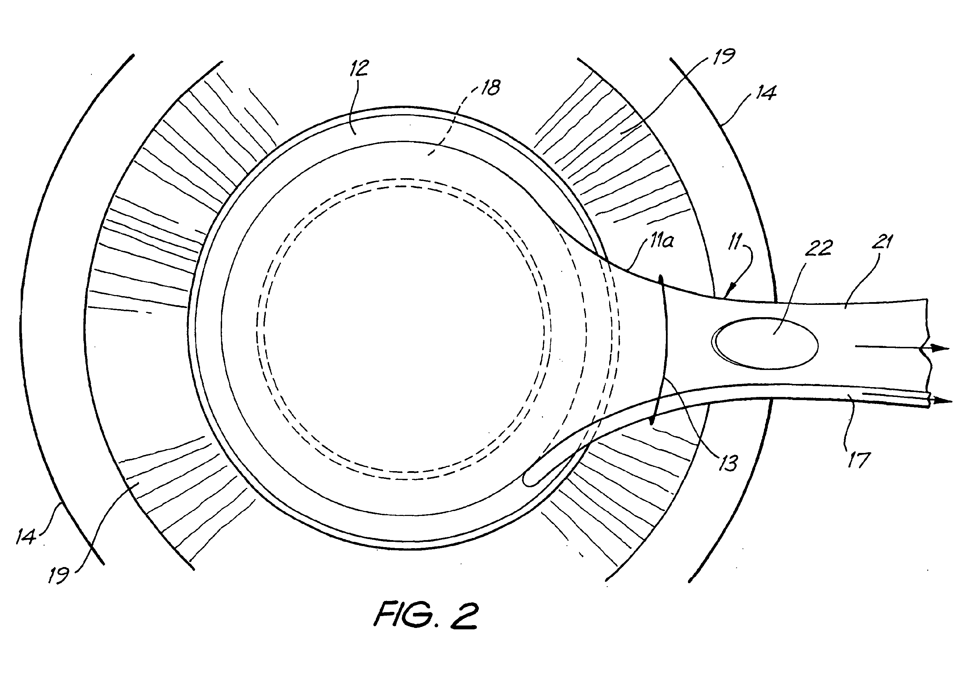

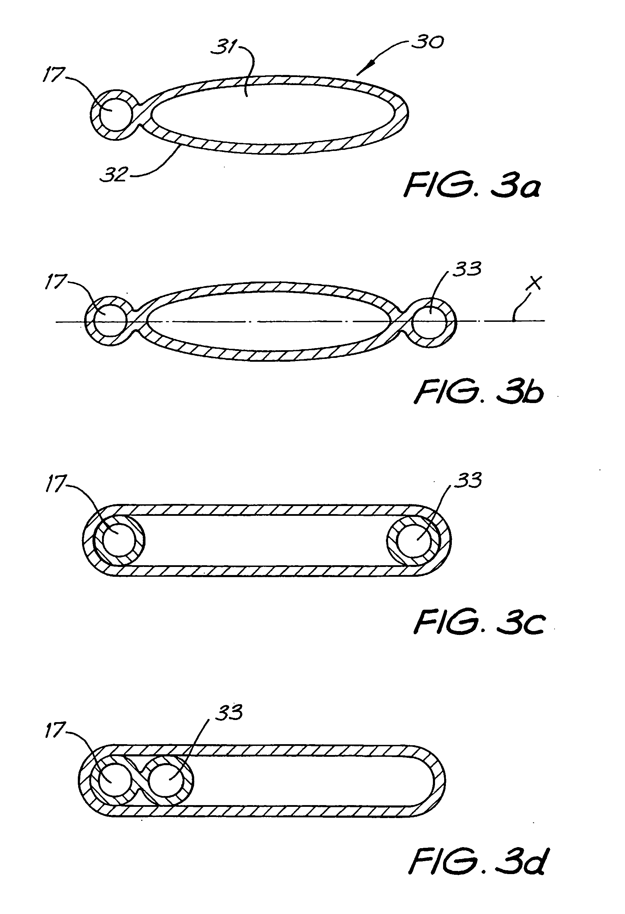

As shown in Fig. 2, the distal portion 11 a of the sleeve 11 and suction ring 12 fit through the corneal incision 13 and the remainder of the sleeve 11 is external to the cornea 14. The external portion 21 of the sleeve 11 has an opening 22 formed in it. The opening 22 allows instrumentation access through the corneal incision 13. The portion of the sleeve 11 which passes through the corneal incision 13 and those areas immediately around it are referred to as the throat of the sleeve. In this instance, the throat has a maximum dimension of about 2.6mm and in the area of the incision should be relatively flat to minimise wound distortion (see Fig. 3). The relatively flat configuration of the throat also provides, in use, some back pressure to the lens capsule, which back pressure allows the capsule to be inflated when fluid is introduced through the sleeve.

As shown in Fig. 3, the throat area 30 can assume various configurations. As shown in Fig. 3a, the throat 30 forms a flat ellipse with the suction tube 17 aligned along the long axis of the ellipse 31 of the throat 30. In this example, the suction tube 17 is joined to the exterior surface 32 of the sleeve 11. Fig. 3b illustrates an example in which a suction tube 17 and another tube 33 are carried on the exterior of the sleeve 11, both aligned with the long axis "X" of the ellipse 31 of the throat. Fig. 3c illustrates an embodiment where the suction tube 17 and the other tube 33 are located on or along the interior of the sleeve 11. In this example, the two tubes 17, 33 are located at opposite ends of the flat throat portion. In Fig. 3d, the suction tube 17 and the other tube 33 are adjacent to one another and located at the same side of the throat.

The other tube 33 referred to in Figs. 3b to 3d can be used for any one of a variety of purposes. In one example, the other tube 33 serves as an alternate or secondary suction tube. In other embodiments, the other tube 33 can be used to either deliver or withdraw fluids (including gases if required) to the interior of the sleeve 11 and thereby into the lens capsule through the surgical opening in the capsule. In some embodiments, it will be preferable to terminate the tube 33 at a location diametrically opposite the point at which the suction tube 17 joins the suction ring 12. In this configuration, the tube 33 can serve as a guide for an insertion rod. Such a rod is inserted into the tube 33 for the purpose of manoeuvring the device 10 though the corneal incision 13 and into position on the capsule 15.

With respect to Figs. 3a to 3d, it will be appreciated that the precise configuration of the sleeve and the throat section of the sleeve are such that the combination of cross section or configuration, wall thickness and sleeve geometry result in a slight but correct degree of back pressure into the lens capsule.

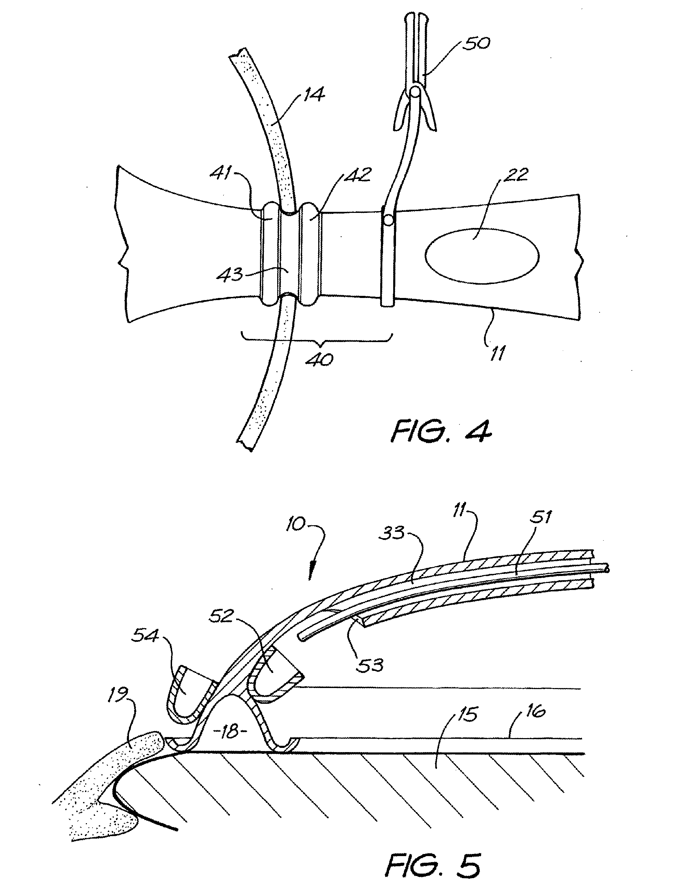

As shown in Fig. 4, the throat area 40 of the sleeve 11 passes through the cornea 14. In order to stabilise the interface between the sleeve 11 and the cornea 14, one or more stabilising ribs 41, 42 are provided around the exterior circumference of the sleeve 11. In one embodiment, a single circumferential rib is provided at a location between the instrument access opening 22 and the point on the sleeve 11 which passes through the cornea 14. The exterior rib 42 prevents the sleeve from being inserted further than the location of the rib 42. In another embodiment, a second and internal rib 41 is provided between the exterior rib 42 and the suction ring 12. A gap 43 is thereby defined between the internal and external ribs 41, 42, the cornea 14 fitting in the gap 43 between the two ribs 41, 42.

The sleeve 11 may be further stabilised with the provision of a stabilising clamp 50. The clamp 50 is affixed to the exterior of the sleeve 11 in the area between the instrument access opening 22 and the point where the sleeve enters the cornea 14.

With or without the provision of stabilising ribs 41, 42 or clamp 50, the instrument opening 22 which is formed on the proximal end of the sleeve 11 remains external to the cornea and allows instruments such as syringes and irrigation/aspiration cannulas to be inserted into the capsule without directly contacting the anterior chamber of the eye.

It will be appreciated that insertion devices such as forceps, a folder or injector system may be optionally provided to ease insertion of the device 10 into the interior chamber.

As shown in Fig. 5, the second tube 33 (see Figs. 3b to 3d) can be used to temporarily carry an insertion rod 51. Preferably, a small cup 52 or other termination is located within the sleeve 11 and adjacent to the distal opening 53 of the tube 33. The insertion rod 51 allows for convenient insertion and positioning of the device and is fully removed prior to use. The cup 52 admits the tip of the positioning rod 51 and prevents the rod from puncturing the sleeve 11. Once the rod 51 is removed, the tube 33 can be used to carry fluids into or out of the interior space of the sleeve. In the alternative, a cup 54 can be fixed to or integrally moulded with the exterior surface of the sleeve 11 so that the positioning rod 51 can be disposed on the exterior of the sleeve 11. Accordingly, the tube 33 may be positioned on the exterior surface (see Fig. 3b) or other means may be provided to temporarily hold the positioning rod 51 prior to its withdrawal.

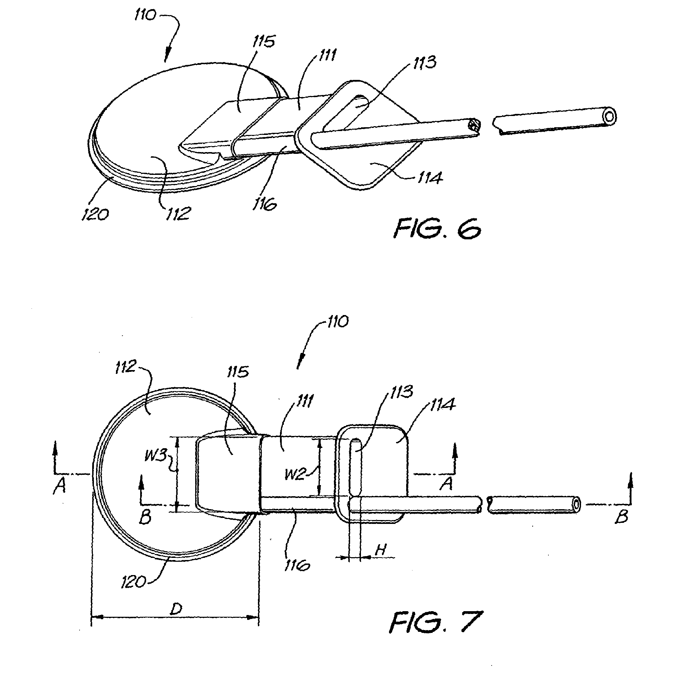

As shown in Fig. 6, a capsule sealing device according to another embodiment of the invention comprises a flexible sleeve 111 which terminates in a vacuum platter 112 and opens onto the underside of the platter 12 thereby creating a sealed fluid channel from the underside through the interior of the sleeve. As previously mentioned, the sleeve 111 and the platter 112 are preferably made from silicone or polyurethane or other soft and flexible materials and the internal surface of the sleeve may be coated with a slippery substance such as a hydro gel. The platter 112 and the distal portion of the sleeve 111 to which it is attached at a predetermined angle (in this instance 20°) must be small enough and flexible enough to pass through an incision in a cornea.

As shown in Figs. 6 to 8, the sleeve 111 incorporates a suction tube 116 and a throat 113. The throat 113 leads from a rectangular (or other shaped) flange 114 to the underside or interior of the platter 112. The somewhat oversized flange acts as a shield, preventing fluid flowing out of the top of the throat from falling back into the eye. The throat 113 provides a passageway through which instruments or fluids or conduits may access the interior of the capsule through the corneal incision. The sleeve 111 and throat 113 are relatively flat to minimise wound distortion. The join 115 between the sleeve 111 and the platter 112 may be slightly thickened or reinforced to provide extra strength in this area.

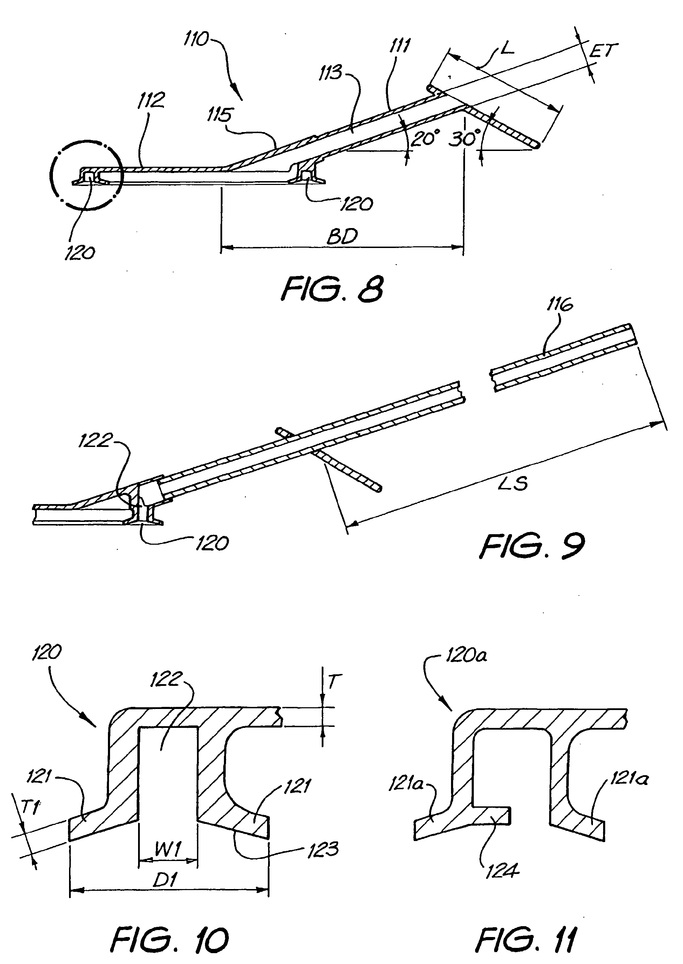

The suction tube 116 is located along one edge of the sleeve 111 and may be moulded into it. The suction tube 116 provides a sealed passageway and fluidic communication with the suction ring or channel 120. The communication between the suction ring or channel 120 and suction tube 116 is shown in Fig. 9. It will be appreciated that the suction tube 116 may be used for a variety of purposes. In one example, the suction tube 116 may serve as a guide for an insertion rod. The throat may also serve this purpose. Such a rod is inserted into the tube 116 for the purpose of manoeuvring the device through a corneal incision and into position on the lens capsule.

As shown in Fig 10, the suction ring 120 which acts as a seal and surrounds the platter 112 comprises a pair of concentric flexible lips 121 which surround an annular channel 122. The flat and flexible undersides 123 of the lips 121 allow the platter 112 to adhere to and seal against the lens capsule with the platter 112 surrounding the capsulorhexis. The suction ring 120 thus forms an inverted "U" cross-section about its entire circumference.

It will be appreciated that the suction platter 112 has been disclosed as disc shaped, but may be fabricated as any convenient plan form such as oval, rectangular or irregular to suit any surface shape or confirmation. Likewise, the suction channel 120 has been disclosed as a ring, but its primary characteristic is merely that it confirms to the shape of the underside of the suction platter 112, regardless of what shape the platter happens to be.

A modified form of the suction ring 120a is shown in Fig. 11. Apart from outwardly directed lips 121a, the ring 120a has an inwardly directed lip 124.

So that the invention may be better understood the following dimensions are intended to serve as examples and not as limitations to the scope of the invention. For example, and with reference to Figs. 7 to 10, the maximum diameter (D) of the platter is about 7mm. The length (L) of the flange is about 3.4mm. The width (W2) of the throat is about 2.35mm. The height (H) of the throat is about 0.43mm. The width (W1) of the central channel 122 of the suction ring 120 is about 0.2mm. The distance (D1) between the inner and outer extremities of the lips 121 is about 1mm. The thickness (T1) of the lips 121 is about 0.1mm. The angle of inclination of the lips 121 with respect to a referenced horizontal plane is about 15° on each side. The thickness (T) of the upper surface of the suction disc 112 is approximately 0.1 mm. The sleeve 111 is inclined with respect to a reference horizontal plain by about 20°. As shown in Fig. 8, the flange 114 is inclined by about 30° to a reference horizontal plane. The suction tube 116 extends by a distance (LS) of about 15mm from the face of the flange 114. The suction tube 116 (which may be insert moulded into the suction disc assembly) has an outside diameter of about 0.63mm and an inside diameter of about 0.3mm. As shown in Fig. 8, the baseline distance (BD) between the centre of the platter 112 and the end of the sleeve 111 is about 7.2mm. The width (W3) of the sleeve 111 is about 3.08mm. The external thickness (ET) of the sleeve is about 0.63mm. The wall thickness of the throat is about 0.1 mm.

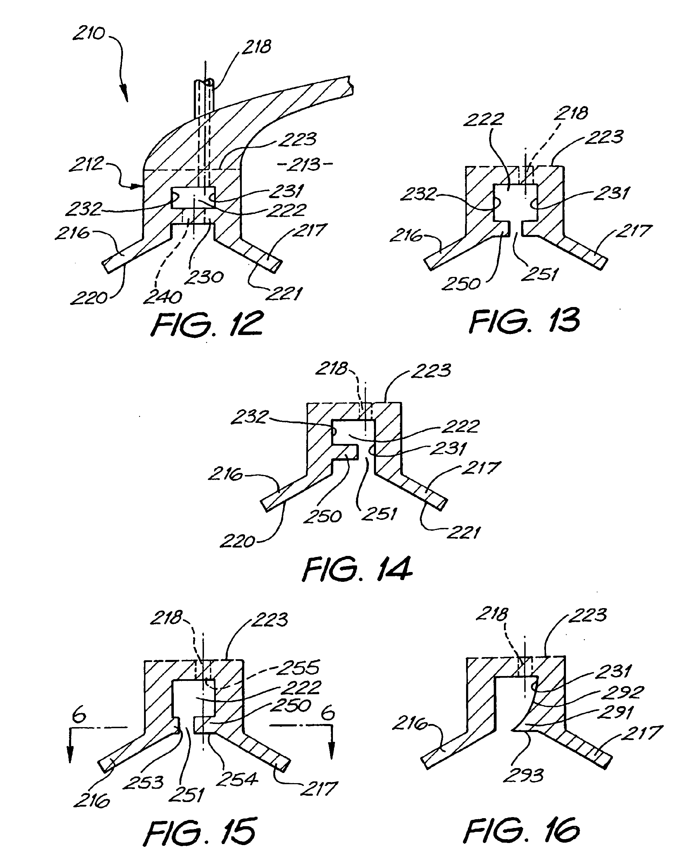

Figs. 12 to 16 are representative of a portion of the cross section of a vacuum sealing device 210 of the general type depicted in Figs. 1 and 6. As such Fig. 12 corresponds generally to the area A of Fig. 1 and illustrates a close up view of a portion of the platters 12 and 212. In this context the term "ring" is not meant to denote a circular shape to the exclusion of other closed curves. As shown in Fig. 12, the platter 212 further comprises an outer edge 216 and an inner edge 217. The bottom surfaces 220, 221 of the outer and inner edges of the platter 212 form body contacting surfaces of the device 210. A manifold 222 is provided above the body contacting surfaces 220, 221. The manifold defines a chamber which follows the contour of the platter 212 and serves to provide uniform vacuum to the entirety of the platter 212 from a source such as the vacuum tube 218 which leads into the manifold 222. For clarity, the platter or suction ring 212 is shown in Fig. 12 as being separate from the remainder of the device by the imaginary or dotted line 223. This same dotted line 223 is used in Figs. 13 to 15 to represent the conceptual boundary between the suction channel or ring 212 and the remainder of the device 210.

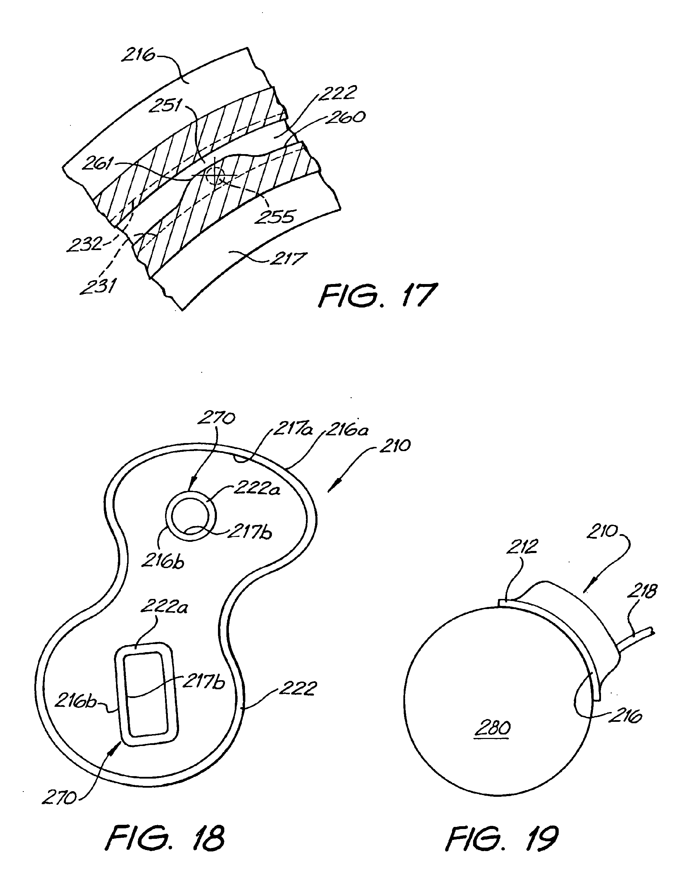

Fig. 12 depicts a continuous membrane barrier or flange 230 which extends between the inner and outer walls 231, 232 of the manifold 222. In some instances, the conformation of the sealing ring 212 will be circular. However, the suction ring 212 need not be exclusively circular and other conformations are contemplated (see Fig 18).

In this example, the barrier flange 230 follows the conformation of the suction channel or ring 212 and but for regular or irregularly spaced openings 240 defines a barrier between the body contacting surfaces 220, 221 and the manifold 222. The openings 240 are sized to permit vacuum pressure to be communicated below the barrier flange 230 while at the same time preventing debris from entering the manifold 222.

As shown in Figs. 13 and 15, the baffle or barrier 250 formed within the manifold 222 may be formed along either or both the inner and outer side walls 231, 232 of the manifold 222. As such, the gap 251 which extends into the manifold 22 may be located along either manifold wall 231, 232 or, as suggested by Fig. 14 centrally of the manifold 222. Fig. 14 also illustrates that the barrier 250 may be located at the lower most extent of the manifold 222.

It will be appreciated that the barrier, eg 240, 250 may be provided as a single flange which follows a side wall (Fig. 13), a pair of flanges which define a gap there between (Fig. 14) or as a continuous but perforated membrane which extends between the side walls (Fig. 12).

Fig. 15 illustrates that a barrier 250 may be comprised of flanges 253, 254 of differing widths. In some embodiments it may be advantageous to provide a flange wide enough to block direct or linear access to the entry port 255 of the vacuum inlet 218. This requires that the entry port 255 and flange 254 be in alignment as depicted in Fig. 16. A variation of this form of alignment is depicted in Fig. 17. In Fig. 17 it can be seen that the width of inner flange 260 (corresponding to flange 254 in Fig. 15) is enlarged only in the area 261 corresponding to the position below the inlet 255.

As shown in Fig. 16, a barrier or flange 291 may be blended 292 into a sidewall 231 rather than project from it like a cantilever. This provides flat bottom 293 with enhanced rigidity.

As shown in Fig. 18, it will be seen that a vacuum sealing device 210 may be provided with a complex peripheral conformation. As shown in this example, the outer extent of the device 210 is still defined by the vacuum seal created by the inner and outer edges of the suction ring 216a, 217a. However, within the closed curve defined by the inner edge 217a, any number of secondary vacuum seals 270 may be provided. Each of the secondary seals 270 has its own vacuum manifold 222a and each is defined by inner and outer edges of its respective suction ring 216b, 217b. In some embodiments it will be preferential to have the various individual vacuum manifolds controlled separately or in discrete groups. As exemplified by Fig. 17, there are practically no limitations to the shape of the primary or secondary seals within the context of the two dimensional plane.

The invention may also be extended to body contacting sealing surfaces which define shapes in three dimensional space rather than just a two dimensional plane. As shown in Fig. 19, a vacuum sealing device 210 may be manufactured so that the suction ring 212 defines a shape which has body contacting surfaces that depart from a two dimensional plane. In this example, suction ring 212 is shown as having been manufactured to conform to a cylindrical surface 280 although it will be understood that the same illustration may be representative of a vacuum sealing device which is manufactured to conform to e.g. a spherical surface 280.

It will be appreciated that the vacuum sealing device 210 disclosed above may be used on a body surface to limit the application of a fluid (or gas) to a defined area. Once such application is LASEK surgery, where alcohol is applied to the cornea of the eye to loosen the epithelium before laser treatment. A similar device to that disclosed here with an approximate 10mm inner seal diameter allows alcohol to be delivered via this sealed system and applied to a limited area of the cornea. This limits the risk of the alcohol solution affecting the surrounding ocular tissues.

## Claims

1. A surgical vacuum sealing device comprising:-one or more suction rings (12, 120);
**characterised in that** the device further comprises a flexible sleeve (11, 111) extending to a distal end to form a flexible platter with the said one or more suction rings (12, 120) in an underside thereof for sealing to a body to define an enclosed surgical area, the, sleeve having a suction tube for communicating a suction or pressure to the one or more suction rings, and having a tube which opens into the underside of the platter and includes an access opening (22) for entry of surgical instruments.

2. A device according to claim 1 wherein the or each suction ring comprises a channel;
wherein a barrier is located within the channel (12, 120), the barrier being attached to one or more walls inside the channel (12, 120).

3. A device according to claim 1 or 2 wherein the suction tube (17, 116) is, along at least a portion, joined to or incorporated into the sleeve (11, 111).

4. A device according to any preceding claim further comprising at least one auxiliary tube (33) carried within or by the sleeve (11, 111), said auxiliary tube (17, 116) having a distal end which communicates with the underside of the platter for the delivery or removal of fluids.

5. A device according to claim 4 further comprising a positioning rod (51) which fits within the auxiliary tube (33).

6. A device according to any preceding claim wherein the sleeve (11, 111) is provided with one or two stabilizing ribs (41, 42) located about an exterior circumference thereof.

7. A device according to any preceding claim wherein the sleeve (11, 111) has a cross-section (30, 40, 113) which is flat shaped so as to minimise wound

8. A device according to any preceding claim formed to be flexible.

9. A device according to claim 8 wherein the sleeve (11, 111) extends from the platter at a predetermined angle to allow it to pass though a corneal incision (13).

10. A device according to claim 8 or 9 wherein the platter is sized to surround a capsulorhexis.

11. A device according to claim 8 or 9 wherein the sleeve (11, 111) is sized to fit within the margin of a pupil.

## Patentansprüche

1. Chirurgische Vakuumabdichtvorrichtung umfassend:
einen oder mehrere Saugring(e) (12, 120)
**gekennzeichnet dadurch, daß** die Vorrichtung des weiteren eine Hülse (11, 111) umfaßt, die sich zu einem distalen Ende erstreckt, um eine flexible Platte mit dem besagten einen oder mehreren Saugring(en) (12, 120) in deren Unterseite zu bilden, um einen Körper abzudichten, um eine geschlossene chirurgische Fläche zu definieren, die Hülse hat eine Saugröhre, um einen Sog oder einen Druck auf den einen oder mehrere Saugring(e) zu übertragen und sie hat eine Röhre, die sich in die Unterseite der Platte öffnet und eine Zugangsöffnung (22) für den Eintritt von chirurgischen Instrumenten einschließt.

2. Vorrichtung nach Anspruch 1, bei der der oder jeder Saugring einen Kanal umfaßt;
wobei eine Barriere innerhalb des Kanals (12, 120) lokalisiert ist, die Barriere ist an eine oder mehrere Wand/Wände innerhalb des Kanals (12,120) angebracht.

3. Vorrichtung nach Anspruch 1 oder 2, bei der die Saugröhre (17, 116) zumindest entlang einem Teilbereich verbunden ist mit der oder inkorporiert in die Hülse (11,111) ist.

4. Vorrichtung nach einem der vorherigen Ansprüche, des weiteren umfassend wenigstens eine Hilfsröhre (33), die innerhalb der oder durch die Hülse (11, 111) getragen wird, die Hilfsröhre (17, 116) hat ein distales Ende, das mit der
Unterseite der : Platte für die Abgabe oder Entfernung von Flüssigkeiten kommuniziert.

5. Vorrichtung nach Anspruch 4, des weiteren umfassend : einen Positionierungsstab (51), der in die Hilfsröhre (33) paßt.

6. Vorrichtung nach einem der vorherigen Ansprüche; bei dem die Hülse (11, 111) mit einer oder zwei Stabilisierungsrippen (41, 42) ausgestattet ist, die um deren äußeren Umfang lokalisiert sind.

7. Vorrichtung nach einem der vorherigen Ansprüche, bei dem die Hülse (11, 111) einen Querschnitt (30, 40, 113) aufweist, der flach gestaltet ist, um eine Wunddistorsion:zu minimieren.

8. Vorrichtung nach einem der vorherigen Ansprüche, die flexibel geformt ist,

9. Vorrichtung nach Anspruch 8, bei der sich die Hülse (11, 111) von der Platte in einem vorherbestimmten Winkel erstreckt, um die Führung durch einen Komealeinschnitt (13) zu ermöglichen.

10. Vorrichtung nach Anspruch 8 oder 9, bei der die Platte so abgemessen ist, daß sie eine Capsulorhexis umschließt.

11. Vorrichtung nach Anspruch 8 oder 9, bei der die Hülse (11, 111) so abgemessen ist, daß si in den Rand einer Pupille passt.

## Revendications

1. Dispositif chirurgical de fermeture étanche sous vide, comprenant :
une ou plusieurs bagues d'aspiration (12, 120),
**caractérisé par le fait que** le dispositif comprend en outre un manchon flexible (11, 111) s'étendant jusqu'à une extrémité distale afin de former un disque flexible avec ladite une ou lesdites plusieurs bagues d'aspiration (12, 120) dans un côté inférieur de celui-ci pour une fermeture étanche sur un corps pour définir une zone chirurgicale fermée, le manchon ayant un tube d'aspiration pour faire communiquer une aspiration ou pression à la ou aux bagues d'aspiration, et ayant un tube qui s'ouvre dans le côté inférieur du disque et comprend une ouverture d'accès (22) pour l'introduction d'instruments chirurgicaux.

2. Dispositif selon la revendication 1, dans lequel la ou chaque bague d'aspiration comprend un canal ;
dans lequel une barrière est située à l'intérieur du canal (12, 120), la barrière étant fixée à une ou plusieurs parois à l'intérieur du canal (12, 120).

3. Dispositif selon l'une des revendications 1 ou 2, dans lequel le tube d'aspiration (17, 116) est, le long d'au moins une partie, réuni à ou incorporé dans le manchon (11, 111).

4. Dispositif selon l'une quelconque des revendications précédentes, comprenant en outre au moins un tube auxiliaire (33) porté à l'intérieur ou par le manchon (11, 111), ledit tube auxiliaire (17, 116) ayant une extrémité distale qui communique avec le côté inférieur du disque pour la distribution ou le retrait de fluides.

5. Dispositif selon la revendication 4, comprenant en outre une tige de positionnement (51) qui s'adapte à l'intérieur du tube auxiliaire (33).

6. Dispositif selon l'une quelconque des revendications précédentes, dans lequel le manchon (11, 111) est doté d'une ou plusieurs nervures de stabilisation (41, 42) situées autour d'une périphérie extérieure de celui-ci.

7. Dispositif selon l'une quelconque des revendications précédentes, dans lequel le manchon (11, 111) a une section transversale (30, 40, 113) qui est de forme plate de façon à rendre minimale la distorsion de la plaie.

8. Dispositif selon l'une quelconque des revendications précédentes, formé pour être flexible.

9. Dispositif selon la revendication 8, dans lequel le manchon (11, 111) s'étend à partir du disque à un angle prédéterminé pour lui permettre de passer à travers une incision cornéenne (13).

10. Dispositif selon l'une des revendications 8 ou 9, dans lequel le disque est dimensionné pour entourer un capsulorhexis.

11. Dispositif selon l'une des revendications 8 ou 9, dans lequel le manchon (11, 111) est dimensionné pour s'adapter à l'intérieur du bord d'une pupille.
